# EUROPEAN PATENT SPECIFICATION

(11) **EP 2 185 155 B1**
(45) Date of publication and mention of the grant of the patent: **04.10.2017**
(21) Application number: 08782602.0
(22) Date of filing: 04.08.2008
(51) Int. Cl.: A61K 31/496, A61P 25/24

(54) **PHARMACEUTICAL COMPOSITIONS CONTAINING DOPAMINE RECEPTOR LIGANDS AND METHODS OF TREATMENT USING DOPAMINE RECPTOR LIGANDS**
PHARMAZEUTISCHE ZUSAMMENSETZUNGEN MIT DOPAMIN-REZEPTOR-LIGANDEN UND BEHANDLUNGSVERFAHREN MIT DOPAMIN-REZEPTOR-LIGANDEN
COMPOSITIONS PHARMACEUTIQUES CONTENANT DES LIGANDS DES RÉCEPTEURS DOPAMINERGIQUES ET PROCÉDÉ DE TRAITEMENT METTANT EN OEUVRE DES LIGANDS DES RÉCEPTEURS DE LA DOPAMINE

(30) Priority: 03.08.2007 US 953694 P
(43) Date of publication of application: 19.05.2010
(73) Proprietor: Richter Gedeon Nyrt., 1103 Budapest (HU)
(72) Inventor: ADHAM, Nika, Englewood Cliffs, NJ 07632 (US); SAMORISKI, Gary, Hillsborough, NJ 08844 (US)
(74) Representative: Hoffmann Eitle
(86) International application number: PCT/US2008/072066
(87) International publication number: WO 2009/020897

(56) References cited:
- WO-A1-2006/082456
- WO-A2-2008/139235
- US-A1- 2006 229 297
- US-A1- 2006 229 297
- US-A1- 2007 042 014
- US-B2- 7 030 122
- HILLERT A ET AL: "Risperidone in the treatment of disorders with a combined psychotic and depressive syndrome--a functional approach.", PHARMACOPSYCHIATRY, vol. 25, no. 5, September 1992 (1992-09), pages 213-217, XP009156770, ISSN: 0176-3679
- JANICAK PHILIP G ET AL: "Risperidone monotherapy versus risperidone or haloperidol plus sertraline for major depression with psychosis: Results of a pilot, double-blind, placebo-controlled trial", BIOLOGICAL PSYCHIATRY, vol. 59, no. 8, Suppl, 1 April 2006 (2006-04-01), page 146S, XP009156754, ELSEVIER SCIENCE, NEW YORK, NY, US ISSN: 0006-3223
- MATHEWS J ET AL: "Antidepressant efficacy of olanzapine as monotherapy in major depressive disorder, without psychosis: A pilot study", PSYCHIATRY RESEARCH: NEUROIMAGING, vol. 146, no. 2, 31 March 2006 (2006-03-31), pages 149-155, XP027998411, ELSEVIER, AMSTERDAM, NL ISSN: 0925-4927, DOI: 10.1016/J.PSCYCHRESNS.2005.08.003 [retrieved on 2006-03-31]
- GYERTYAN I ET AL: "P.3.d.006 Pharmacological profile of RGH-188, a novel dopamine D3/D2 receptor antagonist/partial agonist atypical antipsychotic", EUROPEAN NEUROPSYCHOPHARMACOLOGY, vol. 16, 1 January 2006 (2006-01-01), page S432, XP027969902, ELSEVIER SCIENCE PUBLISHERS BV, AMSTERDAM, NL ISSN: 0924-977X [retrieved on 2006-01-01]
- Thomson Reuters Drug News (formerly DailyDrugNews.com): "Gedeon Richter signs new agreement for antipsychotic RGH-188", Thomson Reuters Integrity , 16 May 2006 (2006-05-16), XP002670062, Retrieved from the Internet: URL:https://integrity.thomson-pharma.com/i ntegrity/xmlxsl/pk_ref_list.xml_show_ficha _ref?p_ref_id=990898 [retrieved on 2012-02-22]
- PAPAKOSTAS ET AL: "Dopaminergic-based pharmacotherapies for depression", EUROPEAN NEUROPSYCHOPHARMACOLOGY, vol. 16, no. 6, 1 August 2006 (2006-08-01) , pages 391-402, XP025110892, ELSEVIER SCIENCE PUBLISHERS BV, AMSTERDAM, NL ISSN: 0924-977X, DOI: 10.1016/J.EURONEURO.2005.12.002 [retrieved on 2006-08-01]
- NUMBERG.: 'Managing Treatment-Emergent Sexual Dysfunction Associated with Serotogenic Antidepressants: Before and After Sildenafil' JOURNAL OF PSYCHIATRIC PRACTICE vol. 7, 2001, page 98, XP008130700

## Description

### FIELD OF THE INVENTION

The present invention relates to cariprazine and pharmaceutically acceptable salts thereof for use in the treatmentof major depressive disorder.

### BACKGROUND OF THE INVENTION

Schizophrenia is a lifelong disabling psychiatric disorder with a reported worldwide prevalence of about 1%, including 3.2 million Americans (see, e.g., Mueser and McGurk, Lancet, 363, 2063-72, 2004). The disorder usually manifests during adolescence or in young adulthood; the cardinal symptoms fall into three domains: positive symptoms, such as delusions and hallucinations, negative symptoms, such as lack of drive and social withdrawal, and cognitive symptoms, such as problems with attention and memory. These lead to social and occupational dysfunction, which inevitably have a profound effect on the family and the place of the affected individual in wider society. In addition to psychiatric symptoms, patients with schizophrenia are at greater risk for medical comorbidities than the general population.

Current guidelines recommend atypical antipsychotics, including risperidone, olanzapine, quetiapine, ziprasidone, and aripiprazole, as first-line treatment for schizophrenia. These drugs can be uniformly characterized by their dual mode of action: in addition to antagonism of the dopamine D₂ receptor, they are also potent inhibitors at the serotonin 5-HT_{2A} receptor.

Although an improvement over the classical neuroleptics, atypical antipsychotics still have shortcomings in the effective management of the disease. In particular, these drugs are associated with a high incidence of side effects (e.g., extrapyramidal symptoms [EPSs] at high dose, sedation, cardiovascular effects such as QTc prolongation, hematologic alterations, effects on sexual function, weight gain, metabolic abnormalities). Furthermore, treatment resistance remains high with 10-30% of patients having little or no response to currently available antipsychotic medications, and up to an additional 30% of patients having only partial treatment response (see, e.g., Lehman et al., Am. J. Psychiatry,161 (2 Suppl), 1-56, 2004). This has led to the common clinical practice of experimental use of high doses of atypicals, antipsychotic polypharmacy, and augmentation with other psychotropic drugs (see, e.g., Zink et al., Eur. Psychiatry, 19:56-58, 2004; Stahl and Grady, Curr. Med. Chem., 11, 313-27, 2004).

Bipolar disorder is a complex, chronic illness causing dramatic mood swings and unusual shifts in energy and behavior, ultimately resulting in functional impairments; it is associated with significant morbidity and mortality. It manifests itself as alterations in mood and energy from euphoria and excitability to depression and psychomotor retardation (Goodwin and Jamison, 1990 (Goodwin FK, Jamison KR. In: Manic-depressive illness. New York: Oxford University Press, 642-647, 1990), and is associated with significant morbidity and mortality. Suicide rates within this population are among the highest of all psychiatric illnesses (Müller-Oerlinghausen et al., Lancet, 359 (9302), 241-7, 2002). Bipolar disorder is treated in phases, with each phase presenting its own set of challenges to the treating physician. Bipolar mania accounts for one in seven psychiatric emergencies. Acute manic and mixed episodes are frequently associated with severe behavioral, physical, functional, and cognitive disturbances, all of which can have important personal and social consequences.

A variety of pharmacological agents are currently available for the management of acute mania, including mood stabilizers, anticonvulsants, and antipsychotics. In recent years, the atypical antipsychotics (eg, olanzapine, risperidone, quetiapine, ziprasidone, aripiprazole) have been approved for mania in bipolar disorder. Compared to conventional agents, the side effect profile of atypical antipsychotics is more favorable. However, the atypicals have been associated with an increased risk of metabolic side effects, including body weight gain, dyslipidemia, glucose intolerance, and type II diabetes. Because of this increased risk, the FDA requires a warning label for diabetes on all atypical antipsychotics. Other side effects commonly associated with currently available treatment options for acute mania in bipolar patients include tremors, psychomotor slowing, cognitive impairment, exacerbation of agitation, nephrotoxicity, altered thyroid function, and sexual dysfunction.

Therefore, despite substantial advances in the pharmacological treatment of bipolar disorder, treatment needs are still not met by currently available therapies and only a low percentage of patients persistently benefit from treatment (Sachs, J. Clin. Psychopharmacol., 23 (3 Suppl 1), S2-8, 2003). A significant percentage of patients do not fully respond to these treatment options and continue to experience subthreshold symptoms and even relapse These drawbacks limit their applicability and result or contribute to patient noncompliance.

Mood disorders, of which major depressive disorder is one of the most common, affect one person in five during their lifetime. The World Health Organization estimates that depression is currently the fourth most important worldwide cause of disability-adjusted life year loss, and that it will become the second most important cause by 2020 (See, e.g., Science, 288, 39-40, 2000). Major depressive disorder is a serious mental disorder that profoundly affects an individual's quality of life. Unlike normal bereavement or an occasional episode of "the blues," MDD causes a lengthy period of gloom and hopelessness, and may rob the sufferer of the ability to take pleasure in activities or relationships that were previously enjoyable. In some cases, depressive episodes seem to be triggered by an obviously painful event, but MDD may also develop without a specific stressor. Research indicates that an initial episode of depression is likely to be a response to a specific stimulus, but later episodes are progressively more likely to start without a triggering event. A person suffering major depression finds job related responsibilities and such other tasks as parenting burdensome and carried out only with great effort. Mental efficiency and memory are affected, causing even simple tasks to be tiring and irritating. Sexual interest dwindles; many people with MDD become withdrawn and avoid any type of social activity. Even the ability to enjoy a good meal or a sound night's sleep is frequently lost; many depressed people report a chronic sense of malaise (general discomfort or unease). For some, the pain and suffering accompanying MDD becomes so unendurable that suicide is viewed as the only option; MDD has the highest mortality rate of any mental disorder.

The condition of an individual suffering from a major depressive disorder is sometimes complicated by the fact that the individual is also suffering from anxiety. Thus in addition to the symptoms of their depressive illness, the patient may show signs of excessive or uncontrolled worry, irritability, feelings of tension, fears, restlessness and insomnia, difficulty in concentrating, and multiple somatic complaints such as pains and aches, twitching, stiffness, myoclonic jerks, tinnitus, blurred vision, hot and cold flushes, etc., all of which add to the individual's social and occupational impairment.

Pharmaceutical treatment of depression is frequently inadequate, with many patients typically not achieving remission, even after several months of treatment. Further, there are high recurrence rates - approximately 85% of patients who achieve remission will suffer another episode of major depression. Finally, many currently available antidepressants are associated with side effects that lead some patients to stop taking their medications at risk of sinking back (further) into depression, and to morbidity in others.

The use of risperidone in the treatment of disorders with a combined psychotic and depressive syndrome is described in Pharmacopsychiat. 25 (1992) pages 213 - 217. XP009156754 describes risperidone monotherapy vs risperidone or haloperidol plus sertraline for major depression with psychosis. Antidepressant efficacy of olanzapine as monotherapy in major depressive disorder without psychosis is the subject of a publication of J. Mathews et al., Psychiatry Research: Neuroimaging 146 (2006, pages 149 - 155).

Thus, many of today's drugs are neither completely safe nor completely tolerable for many patients. There is, therefore, an existing and continual need for new formulations and new methods to treat conditions such as schizophrenia, depression, major depressive disorder, acute mania and bipolar disorder, where the pharmaceuticals are effective for a broader rage of patients (particularly treatment-resistant patients), that are safe and more tolerable, or that complement the efficacy of existing drugs.

### SUMMARY OF THE INVENTION

The present invention relates to trans-4={2-[4-(2,3-dichlorophenyl)-piperazin-1-yl]-ethyl}-N,N-dimethylcarbamoyl-cyclohexylamine, or a pharmaceutically acceptable salt thereof, for use in the treatment of a major depressive disorder.

### BRIEF DESCRIPTION OF THE FIGURES

Figure 1 shows the effects of chronic treatment with vehicle and imipramine on the consumption of 1% sucrose solution in controls (open symbols) and in animals exposed to chronic mild stress (closed symbols).
Figure 2 shows the effects of chronic treatment with vehicle and cariprazine hydrochloride on the consumption of 1% sucrose solution in controls (open symbols) and in animals exposed to chronic mild stress (closed symbols).
Figure 3 shows the effects of chronic treatment with vehicle and escitalopram oxalate on the consumption of 1% sucrose solution in controls (open symbols) and in animals exposed to chronic mild stress (closed symbols).
Figure 4 shows the effects of chronic treatment with vehicle, escitalopram oxalate and cariprazine hydrochloride on the consumption of 1% sucrose solution in controls (open symbols) and in animals exposed to chronic mild stress (closed symbols).
Figure 5 shows the effects of chronic treatment with vehicle, escitalopram oxalate and cariprazine hydrochloride administered alone or in combination with escitalopram oxalate on the consumption of 1% sucrose solution in animals exposed to chronic mild stress.
Figure 6 shows the effects of chronic treatment with vehicle, escitalopram oxalate and cariprazine hydrochloride administered alone or in combination with escitalopram oxalate on the consumption of 1% sucrose solution in animals exposed to chronic mild stress.
Figure 7 shows the effects of chronic treatment with cariprazine hydrochloride alone, escitalopram oxalate alone or cariprazine hydrochloride in combination with escitalopram oxalate on the consumption of 1% sucrose solution in animals exposed to chronic mild stress.

### DETAILED DESCRIPTION OF THE INVENTION

The present invention relates to cariprazine and pharmaceutically acceptable salts thereof for use in the treatment of major depressive disorder.

Cariprazine is a compound of formula (I) and the synthesis of compounds of formula (I) is disclosed in, e.g., U.S. Patent Publication No. 2006/0229297. The compounds of formula (I) are orally active and very potent dopamine D₃/D₂ receptor antagonists, which bind with significantly higher potentcy to D₃ than D₂ receptors. The D₃ receptor antagonism is about one order of magnitude greater than the D₂ receptor antagonism, which is believed to counteract some of the extrapyramidal side effects produced by D₂ receptor antagonists. In addition to the increased relative affinity for dopamine D₃ to D₂, compounds of formula (I) have a low potency at other receptor sites such as the 5-HT_{2C}, histamine H₁, and adrenergic receptor sites, which suggest a lower potential for side effects such as EPSs and body weight gain.

In a preferred embodiment the compound for use in the treatment of major depressive disorder is trans-4-{2-[4-(2,3-dichlorophenyl)-piperazin-1-yl]-ethyl}-N,N-dimethylcarbamoyl-cyclohexylamine hydrochloride (cariprazine hydrochloride).

Pharmaceutically acceptable salts include those obtained by reacting the main compound, functioning as a base with an inorganic or organic acid to form a salt, for example, salts of hydrochloric acid, sulfuric acid, phosphoric acid, methane sulfonic acid, camphor sulfonic acid, oxalic acid, maleic acid, succinic acid, citric acid, formic acid, hydrobromic acid, benzoic acid, tartaric acid, fumaric acid, salicylic acid, mandelic acid, and carbonic acid. Pharmaceutically acceptable salts also include those in which the main compound functions as an acid and is reacted with an appropriate base to form, e.g., sodium, potassium, calcium, magnesium, ammonium, and choline salts. Those skilled in the art will further recognize that acid addition salts may be prepared by reaction of the compounds with the appropriate inorganic or organic acid via any of a number of known methods. Alternatively, alkali and alkaline earth metal salts can be prepared by reacting the compounds of the invention with the appropriate base via a variety of known methods.

The following are further examples of acid salts that can be obtained by reaction with inorganic or organic acids: acetates, adipates, alginates, citrates, aspartates, benzoates, benzenesulfonates, bisulfates, butyrates, camphorates, digluconates, cyclopentanepropionates, dodecylsulfates, ethanesulfonates, glucoheptanoates, glycerophosphates, hemisulfates, heptanoates, hexanoates, fumarates, hydrobromides, hydroiodides, 2-hydroxy-ethanesulfonates, lactates, maleates, methanesulfonates, nicotinates, 2-naphthalenesulfonates, oxalates, palmoates, pectinates, persulfates, 3-phenylpropionates, picrates, pivalates, propionates, succinates, tartrates, thiocyanates, tosylates, mesylates and undecanoates.

For example, the pharmaceutically acceptable salt can be a hydrochloride salt, a hydrobromide salt or an oxalate salt

Some of the compounds useful in the present disclosure can exist in different polymorphic forms. As known in the art, polymorphism is an ability of a compound to crystallize as more than one distinct crystalline or "polymorphic" species. The use of such polymorphs is within the scope of the present disclosure. Some of the compounds useful in the present disclosure can exist in different solvate forms. Solvates of the compounds of the invention may also form when solvent molecules are incorporated into the crystalline lattice structure of the compound molecule during the crystallization process. For example, suitable solvates include hydrates, e.g., monohydrates, dihydrates, sesquihydrates, and hemihydrates. The use of such solvates is within the scope of the present disclosure. One of ordinary skill in the art will recognize that compounds of Formula I can exist in different tautomeric and geometrical isomeric forms. All of these compounds, including *cis* isomers, *trans* isomers, diastereomic mixtures, racemates, nonracemic mixtures of enantiomers, substantially pure, and pure enantiomers, are within the scope of the present disclosure. Substantially pure enantiomers contain no more than 5% w/w of the corresponding opposite enantiomer, such as no more than 2%, for example no more than 1%.

The optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, dibenzoyltartaric, ditoluoyltartaric and camphorsulfonic acid. Mixtures of diastereoisomers can be separated into their individual diastereomers on the basis of their physical and/or chemical differences by methods known to those skilled in the art, for example, by chromatography or fractional crystallization. The optically active bases or acids are then liberated from the separated diastereomeric salts. A different process for separation of optical isomers involves the use of chiral chromatography (e.g., chiral HPLC columns), with or without conventional derivation, optimally chosen to maximize the separation of the enantiomers. Suitable chiral HPLC columns are manufactured by Diacel, e.g., Chiracel OD and Chiracel OJ among many others, all routinely selectable. Enzymatic separations, with or without derivitization, are also useful. The optically active compounds of Formula I can likewise be obtained by utilizing optically active starting materials in chiral synthesis processes under reaction conditions which do not cause racemization.

In addition, one of ordinary skill in the art will recognize that the compounds of Formula I can be used in different enriched isotopic forms, e.g., enriched in the content of ²H, ³H, ¹¹C, ¹³C and/or ¹⁴C. In one particular embodiment, the compounds are deuterated. Such deuterated forms can be made the procedure described in U.S. Patent Nos. 5,846,514 and 6,334,997. As described in U.S. Patent Nos. 5,846,514 and 6,334,997, deuteration can improve the efficacy and increase the duration of action of drugs.

Deuterium substituted compounds can be synthesized using various methods such as described in: Dean, Dennis C.; Editor. Recent Advances in the Synthesis and Applications of Radiolabeled Compounds for Drug Discovery and Development. [In: Curr., Pharm. Des., 2000; 6(10)] (2000), 110 pp. CAN 133:68895 AN 2000:473538 CAPLUS; Kabalka, George W.; Varma, Rajender S. The synthesis of radiolabeled compounds via organometallic intermediates. Tetrahedron (1989), 45(21), 6601-21, CODEN: TETRAB ISSN:0040-4020. CAN 112:20527 AN 1990:20527 CAPLUS; and Evans, E. Anthony. Synthesis of radiolabeled compounds, J. Radioanal. Chem. (1981), 64(1-2), 9-32. CODEN: JRACBN ISSN:0022-4081, CAN 95:76229 AN 1981:476229 CAPLUS.

### Dosage Forms

Numerous standard references are available that describe procedures for preparing various formulations suitable for administering the compounds according to the invention. Examples of potential formulations and preparations are contained, for example, in the Handbook of Pharmaceutical Excipients, American Pharmaceutical Association (current edition); Pharmaceutical Dosage Forms: Tablets (Lieberman, Lachman and Schwartz, editors) current edition, published by Marcel Dekker, Inc., as well as Remington's Pharmaceutical Sciences (Arthur Osol, editor), 1553-1593 (current edition).

The mode of administration and dosage forms is closely related to the therapeutic amounts of the compounds or compositions which are desirable and efficacious for the given treatment application.

Suitable dosage forms include oral, rectal, sub-lingual, mucosal, nasal, ophthalmic, subcutaneous, intramuscular, intravenous, transdermal, spinal, intrathecal, intra-articular, intra-arterial, sub-arachinoid, bronchial, lymphatic, and intra-uterille administration, and other dosage forms for systemic delivery of active ingredients. Formulations suitable for oral administration are preferred.

To prepare such pharmaceutical dosage forms, the active ingredient, is typically mixed with a pharmaceutical carrier according to conventional pharmaceutical compounding techniques. The carrier may take a wide variety of forms depending on the form of preparation desired for administration.

In preparing the compositions in oral dosage form, any of the usual pharmaceutical media may be employed. Thus, for liquid oral preparations, such as, for example, suspensions, elixirs and solutions, suitable carriers and additives include water, glycols, oils, alcohols, flavoring agents, preservatives and coloring agents. For solid oral preparations such as, for example, powders, capsules and tablets, suitable carriers and additives include starches, sugars, diluents, granulating agents, lubricants, binders and disintegrating agents. Due to their ease in administration, tablets and capsules represent the most advantageous oral dosage unit form. If desired, tablets may be sugar coated or enteric coated by standard techniques.

For parenteral formulations, the carrier will usually comprise sterile water, though other ingredients, for example, ingredients that aid solubility or for preservation, may be included. Injectable solutions may also be prepared in which case appropriate stabilizing agents may be employed.

In some applications, it may be advantageous to utilize the active agent in a "vectorized" form, such as by encapsulation of the active agent in a liposome or other encapsulant medium, or by fixation of the active agent, e.g., by covalent bonding, chelation, or associative coordination, on a suitable biomolecule, such as those selected from proteins, lipoproteins, glycoproteins, and polysaccharides.

Treatment methods of the present invention using formulations suitable for oral administration may be presented as discrete units such as capsules, cachets, tablets, or lozenges, each containing a predetermined amount of the active ingredient as a powder or granules. Optionally, a suspension in an aqueous liquor or a non-aqueous liquid may be employed, such as a syrup, an elixir, an emulsion, or a draught.

A tablet may be made by compression or molding, or wet granulation, optionally with one or more accessory ingredients. Compressed tablets may be prepared by compressing in a suitable machine, with the active compound being in a free-flowing form such as a powder or granules which optionally is mixed with, for example, a binder, disintegrant, lubricant, inert diluent, surface active agent, or discharging agent. Molded tablets comprised of a mixture of the powdered active compound with a suitable carrier may be made by molding in a suitable machine.

A syrup may be made by adding the active compound to a concentrated aqueous solution of a sugar, for example sucrose, to which may also be added any accessory ingredient(s). Such accessory ingredient(s) may include flavorings, suitable preservative, agents to retard crystallization of the sugar, and agents to increase the solubility of any other ingredient, such as a polyhydroxy alcohol, for example glycerol or sorbitol.

Formulations suitable for parenteral administration usually comprise a sterile aqueous preparation of the active compound, which preferably is isotonic with the blood of the recipient (e.g., physiological saline solution). Such formulations may include suspending agents and thickening agents and liposomes or other microparticulate systems which are designed to target the compound to blood components or one or more organs. The formulations may be presented in unit-dose or multi-dose form.

Parenteral administration may comprise any suitable form of systemic delivery or delivery directly to the CNS. Administration may for example be intravenous, intra-arterial, intrathecal, intramuscular, subcutaneous, intramuscular, intra-abdominal (e.g., intraperitoneal), etc., and may be effected by infusion pumps (external or implantable) or any other suitable means appropriate to the desired administration modality.

Nasal and other mucosal spray formulations (e.g. inhalable forms) can comprise purified aqueous solutions of the active compounds with preservative agents and isotonic agents. Such formulations are preferably adjusted to a pH and isotonic state compatible with the nasal or other mucous membranes. Alternatively, they can be in the form of finely divided solid powders suspended in a gas carrier. Such formulations may be delivered by any suitable means or method, e.g., by nebulizer, atomizer or metered dose inhaler. Formulations for rectal administration may be presented as a suppository with a suitable carrier such as cocoa butter, hydrogenated fats, or hydrogenated fatty carboxylic acids.

Transdermal formulations may be prepared by incorporating the active agent in a thixotropic or gelatinous carrier such as a cellulosic medium, e.g., methyl cellulose or hydroxyethyl cellulose, with the resulting formulation then being packed in a transdermal device adapted to be secured in dermal contact with the skin of a wearer.

In addition to the aforementioned ingredients, formulations of this invention may further include one or more accessory ingredient(s) selected from diluents, buffers, flavoring agents, binders, disintegrants, surface active agents, thickeners, lubricants and preservatives (including antioxidants). The formulations of the present invention can have immediate release, sustained release, delayed-onset release or any other release profile known to one skilled in the art.

The active ingredients present in the composition can normally be administered in a combined daily dosage regimen (for an adult patient) of, for example, an oral dose between about 0.1 mg and about 500 mg, such as between about 1 mg and about 400 mg, e.g. between about 10 mg and about 250 mg or an intravenous, subcutaneous, or intramuscular dose of between about 0.1 mg and about 100 mg, such as between about 0.1 mg and about 50 mg, e.g. between about 1 and about 25 mg.

In certain embodiments, the pharmaceutical composition includes about 0.05 mg, about 0.1 mg, about 0.2 mg, about 0.25 mg, about 0.3 mg, about 0.4 mg, about 0.5 mg, about 0.75 mg, about 1 mg, about 1.5 mg, about 2 mg, about 2.5 mg, about 3 mg, about 3.5 mg, about 4 mg, about 4.5 mg, about 5 mg, about 5.5 mg, about 6 mg, about 6.5 mg, about 7 mg, about 7.5 mg, about 8 mg, about 8.5 mg, about 9 mg, about 9.5 mg, about 10 mg, about 10.5 mg, about 11 mg, about 11.5 mg, about 12.0 mg, about 12.5 mg, about 13.0 mg, about 13.5 mg, about 14.0 mg, about 14.5 mg or about 15.0 mg of cariprazine or pharmaceutically acceptable salt thereof (e.g., cariprazine hydrochloride).

For example, the pharmaceutical composition includes about 0.1 mg, about 0.25 mg, about 0.5 mg, about 1 mg, about 1.5 mg, about 2 mg, about 2.5 mg, about 3 mg, about 5 mg, about 6 mg, about 7.5 mg, about 9 mg, about 12.5 mg or about 15.0 mg of cariprazine or pharmaceutically acceptable salt thereof (e.g., cariprazine hydrochloride).

In yet further embodiments, cariprazine or pharmaceutically acceptable salt thereof is present in the composition in an amount which ranges between any two of these dosage amounts (e.g., between about 0.1 mg and about 15 mg, between about 0.5 mg and about 12.5 mg, between about 1.5 mg and about 6 mg, between about 6 mg and about 12.5 mg).

The desired dose may be administered as one or more daily sub dose(s) administered at appropriate time intervals throughout the day, or alternatively, in a single dose, for example, for morning or evening administration. For example, the daily dosage may be divided into one, into two, into three, or into four divided daily doses.

The duration of the treatment may be decades, years, months, weeks, or days, as long as the benefits persist.

### Methods of Treatment

The present invention is directed to cariprazine or pharmaceutically acceptable salts thereof for use in the treatment of major depressive disorder, e.g. in the treatment of resistant major depressive disorder.

Dysfunction of the dopaminergic neurotransmitter system is involved in the pathology of several neuropsychiatric and neurodegenerative disorders, such as schizophrenia, drug abuse and Parkinson's disease, respectively. The effect of dopamine is mediated via at least five distinct dopamine receptors belonging to the D₁ - (D₁, D₅) or the D₂ - (D₂, D₃, D₄) families. D₃ receptors have been shown to have characteristic distribution in the cerebral dopaminergic systems. Namely, high densities were found in certain limbic structures, such as nucleus accumbens and islands of Calleja. Therefore, preferential targeting of the D₃ receptors may be a promising approach for more selective modulation of dopaminergic functions and consequently for successful therapeutic intervention in several abnormalities, such as schizophrenia, emotional or cognitive dysfunctions and addiction (see, e.g., Sokoloff, P. et al.: Nature, 1990, 347, 146; Schwartz, J. C., et al.: Clin. Neuropharmacol. 1993, 16, 295; Levant, B.: Pharmacol. Rev. 1997, 49, 231), addiction (see, e.g., Pilla, C. et al.: Nature 1999, 400, 371) and Parkinson's disease (see, e.g., Levant, B. et al.: CNS Drugs 1999, 12, 391) or pain (see, e.g., Levant, B. et al.: Neurosci. Lett. 2001, 303, 9).

The dopamine D₂ receptors are widely distributed in the brain and are known to be involved in numerous physiological functions and pathological states. D₂ antagonists are widely used drugs as antipsychotics, for example. However, it is also well known that massive antagonism of the D₂ receptors leads to unwanted side-effects such as extrapyramidal motor symptoms, psychomotor sedation or cognitive disturbances. These side effects seriously restrict the therapeutic utilization of D₂ antagonist compounds. (Wong A. H. C. et al.: Neurosci. Biobehav. Rev. 2003, 27, 269.)

The present invention relates to a compound of Formula (I): 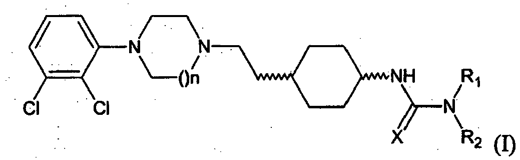 wherein
R₁ and R₂ are each, independently, hydrogen, alkyl, alkenyl, aryl, cycloalkyl or aroyl,
or R₁ and R₂ form a heterocyclic ring with the adjacent nitrogen atom;
X is 0 or S;
n is 1 or 2;
and/or geometric isomers and/or stereoisomers and/or diastereomers and/or salts and/or hydrates and/or solvents and/or polymorphs thereof, for use in the treatment of major depressive disorder, wherein
the compound of formula (I) is trans-4-{2-[4-(2,3-dichlorophenyl)-piperazin-1-yl]-ethyl}-N,N-dimethylcarbamoyl-cyclohexylamine (INN: cariprazine), or a pharmaceutically acceptable salt thereof. For example, the compound of formula (I) is trans-4-{2-[4-(2,3-dichlorophenyl)-piperazin-1-yl]-ethyl}-N,N-dimethylcarbamoyl-cyclohexylamine hydrochloride (cariprazine hydrochloride).

For the treatment of major depressive disorder, cariprazine or pharmaceutically acceptable salt thereof (e.g., cariprazine hydrochloride) can normally be administered in a daily dosage regimen (for an adult patient) of, for example, an oral dose between about 0.1 mg and about 500 mg, such as between about 1 mg and about 400 mg, e.g. between about 10 mg and about 250 mg or an intravenous, subcutaneous, or intramuscular dose of between about 0.1 mg and about 100 mg, such as between about 0.1 mg and about 50 mg, e.g. between about 1 and about 25 mg.

In certain embodiments, cariprazine or pharmaceutically acceptable salt thereof (e.g., cariprazine hydrochloride) administered to treat major depressive disorder is about 0.05 mg, about 0.1 mg, about 0.2 mg, about 0.25 mg, about 0.3 mg, about 0.4 mg, about 0.5 mg, about 0.75 mg, about 1 mg, about 1.5 mg, about 2 mg, about 2.5 mg, about 3 mg, about 3.5 mg, about 4 mg, about 4.5 mg, about 5 mg, about 5.5 mg, about 6 mg, about 6.5 mg, about 7 mg, about 7.5 mg, about 8 mg, about 8.5 mg, about 9 mg, about 9.5 mg, about 10 mg, about 10.5 mg, about 11 mg, about 11.5 mg, about 12.0 mg, about 12.5 mg, about 13.0 mg, about 13.5 mg, about 14.0 mg, about 14.5 mg or about 15.0 mg. For example, the compound of formula (I) or pharmaceutically acceptable salt thereof (e.g., cariprazine hydrochloride), is administered to treat depression in an amount of about 0.1 mg, about 0.25 mg, about 0.5 mg, about 1 mg, about 1.5 mg, about 2 mg, about 2.5 mg, about 3 mg, about 5 mg, about 6 mg, about 7.5 mg, about 9 mg, about 12.5 mg or about 15.0 mg.

In yet further embodiments, the amount of cariprazine, or a pharmaceutically acceptable salt thereof (e.g., cariprazine hydrochloride) administered to treat major depressive disorder ranges between any two of these dosage amounts (e.g., between about 0.1 mg and about 15 mg, between about 0.5 mg and about 12.5 mg, between about 1.5 mg and about 6 mg, between about 6 mg and about 12.5 mg).

In further embodiments, cariprazine is administered to treat major depressive disorder in a daily dosage amount of about 0.01 mg/kg to about 10 mg/kg, e.g., about 0.1 mg/kg to about 1 mg/kg (such as about 0.03 mg/kg, about 0.065 mg/kg, about 0.1 mg/kg, about 0.25 mg/kg, about 0.3 mg/kg or about 1 mg/kg).

### Definitions

The term "escitalopram" as used herein includes 1-[3-(dimethyl-amino)propyl]-1-(p-fluorophenyl)-5-phthalancarbonitrile preferably containing less than 3,2, 1, 0.5, or 0.2% by weight of its R-enantiomer (based on 100% total weight of 1-[3-(dimethyl-amino)propyl]-1-(p-fluorophenyl)-5-phthalancarbonitrile), i.e., S-citalopram having an enantiomeric purity (by weight) of 97, 98, 99, 99.5, or 99.8%. Preferred pharmaceutically acceptable salts of escitalopram include, but are not limited to, escitalopram oxalate and escitalopram hydrobromide. The term "escitalopram" also includes polymorphs, hydrates, solvates, and amorphous forms of escitalopram and its pharmaceutically acceptable salts. Crystals of escitalopram oxalate and escitalopram hydrobromide such as those described in International Publication No. WO 03/011278 and U.S. Patent Application Publication Nos. 2004/0167209, 2005/019653 and 2005/0197388.

The term "pharmaceutically acceptable" means biologically or pharmacologically compatible for *in vivo* use in animals or humans, and preferably means approved by a regulatory agency of the Federal or a state government or listed in the U.S. Pharmacopeia or other generally recognized pharmacopeia for use in animals, and more particularly in humans.

The term "schizophrenia" is intended to include the group of mental disorders characterized by disruptions in thinking and perception, and includes schizophrenia (and all its subtypes; paranoid, catatonic, disorganized, residual, undifferentiated) and other psychotic disorders (as per Diagnostic and Statistical Manual for Mental Disorders, Fourth Edition, Washington, D.C (1994): American Psychiatric Association, or The ICD-10 Classification of Mental and Behavioural Disorders: Clinical Descriptions and Diagnostic Guidelines, Geneva (1992): World Health Organization) such as schizophreniform and schizoaffective disorders, brief psychotic disorder, etc.

In a clinical evaluation, schizophrenia is commonly marked by "positive symptoms" such as hallucinations (especially auditory hallucination which are usually experienced as voices), disorganized thought processes and delusions as well as "negative symptoms" which include affective flattening, alogia, avolition, and anhedonia.

The term "the negative symptoms of schizophrenia" refer to a class of symptoms of schizophrenia which can be considered to reflect a 'loss' in functional, directed thought or activity. Negative symptoms of schizophrenia are well known in the art, and include affective flattening (characterized by, for example, an immobile and/or unresponsive facial expression, poor eye contact and reduced body language), alogia ('poverty of speech' or brief, laconic and/or empty replies), avolition (characterized by a reduced or absent ability to initiate and carry out goal-directed activities), anhedonia (loss of interest or pleasure), asocialty (reduced social drive and interaction), apathy and other negative symptoms known to those of skill in the art. The negative symptoms of schizophrenia may be assessed using any methodology known in the art including, but not limited to, the Brief Psychiatric Rating Scale (BPRS), and the Positive and Negative Symptom Scale (PANSS). The BPRS and PANSS have subscales or factors that can be used to measure negative symptoms. Other scales have been designed to address specifically negative symptoms: For example the Scale for the Assessment of Negative Symptoms (SANS), the Negative Symptoms Assessment (NSA) and the Schedule for the Deficit Syndrome (SDS). Subscales of the BPRS and PANSS may also be used to assess positive symptoms, although methods for specifically assessing positive symptoms are also available (e.g., the Scale for the Assessment of Positive Symptoms, or SAPS).

The term "cognitive deficits associated with schizophrenia" refers to cognitive deficits in schizophrenia patients. Cognitive impairment in schizophrenia is a core feature of the illness (i.e. not a result of treatment or clinical symptoms). Cognitive deficits include, but are not limited to deficits of attention/vigilance, working memory, verbal learning and memory, visuospatial memory, reasoning/problem solving and social cognition. There are numerous neuropsychological tests used to measure cognitive deficits in schizophrenia, such as the Wisconsin Card Sorting Test (WCST).

The terms "treat," "treatment," and "treating" refer to one or more of the following:
(a) relieving or alleviating at least one symptom of a disorder in a subject, including for example, allergic and inflammatory disorders, such as asthma and COPD;
(b) relieving or alleviating the intensity and/or duration of a manifestation of a disorder experienced by a subject including, but not limited to, those that are in response to a given stimulus (e.g., pressure, tissue injury, cold temperature, etc.);
(c) arresting, delaying the onset (i.e., the period prior to clinical manifestation of a disorder) and/or reducing the risk of developing or worsening a disorder.

The term "mood disorder" as used herein includes the mood disorders specified in the DSM-IV-TR, including, but not limited to, depressive disorders, such as major depressive disorder.

Patients suffering from "treatment resistant depression" include (1) those who fail to respond to standard doses (i.e., significantly superior to placebo in double-blind studies) of antidepressants (such as SSRIs) administered continuously for a minimum duration of 6 weeks, and (2) those who fail to respond to standard doses of an antidepressant (such as an SARI) (monotherapy) administered continuously for a minimum duration of 12 weeks. One criteria for determining whether a patient's depression is treatment resistant to an antidepressant is if a Clinical Global Impression-Improvement (CGI-I) score of 1 (very much improved) or 2 (much improved) is not achieved by the end of a 6, 8, or 12 week trial. The CGI-I scale is defined in Guy, W. (ed.): ECDEU Assessment Manual for Psychopharmacology, Revised, DHEW Pub. No. (ADM) 76-338, Rockville, MD, National Institute of Mental Health, 1976.

An "effective amount" means the amount of a composition according to the invention that, when administered to a patient for treating a state, disorder or condition is sufficient to effect such treatment. The "effective amount" will vary depending on the active ingredient, the state, disorder, or condition to be treated and its severity, and the age, weight, physical condition and responsiveness of the mammal to be treated.

A subject or patient in whom administration of the therapeutic compound is an effective therapeutic regimen for a disease or disorder is preferably a human, but can be any animal, including a laboratory animal in the context of a trial or screening or activity experiment. Thus, as can be readily appreciated by one of ordinary skill in the art, the methods, compounds and compositions of the present invention are particularly suited to administration to any animal, particularly a mammal, and including, but by no means limited to, humans, domestic animals, such as feline or canine subjects, farm animals, such as but not limited to bovine, equine, caprine, ovine, and porcine subjects, wild animals (whether in the wild or in a zoological garden), research animals, such as mice, rats, rabbits, goats, sheep, pigs, dogs, cats, etc., avian species, such as chickens, turkeys, songbirds, etc., i.e., for veterinary medical use.

The term "about" or "approximately" means within an acceptable error range for the particular value as determined by one of ordinary skill in the art, which will depend in part on how the value is measured or determined, *i.e*., the limitations of the measurement system. For example, "about" can mean within 1 or more than 1 standard deviations, per practice in the art. Alternatively, "about" with respect to the compositions can mean plus or minus a range of up to 20%, preferably up to 10%, more preferably up to 5%.

### EXAMPLES

The following examples are merely illustrative of the present invention.

### Reference Example 1: Dual-Probe Microdialysis Analysis of Acetylcholine, Dopamine and Serotonin in the Frontal Cortex of Freely-Moving Rats

A dual-probe microdialysis study was conducted to determine the effects of oral administration of cariprazine hydrochloride, alone and in combination with escitalopram oxalate (administered subcutaneously), on extracellular concentrations of acetylcholine (ACh), dopamine (DA) and serotonin (5-HT) in the frontal cortex of freely-moving rats.

### Animals and Environment

Experiments were carried out in male Sprague Dawley rats (250-350 g body weight; Charles River, UK). Animals were housed in groups of six on a 12 h/12 h light/dark cycle (lights on at 07.30 h), at an ambient temperature of 21±2°C and 55±20% humidity. Food and water were available *ad libitum.* Animals were allowed to acclimatise to these conditions for at least 5 days prior to the study.

### Surgery

Rats were anaesthetised with isoflurane (5% to induce, 2% to maintain) in an O₂/N₂O (1 litre/min each) mixture delivered via an anaesthetic unit (St Bernard Medical Services, UK). Concentric microdialysis probes with 2 mm exposed polyarylethersulphone (PAES) membrane tip (CMA) were stereotaxically implanted bilaterally into the prefrontal cortex (coordinates : AP: +3.2 mm; L: +/-2.5 mm relative to bregma; V: -4.0 mm relative to the skull surface; taken from the stereotaxic atlas of Paxinos and Watson (Paxinos G, Watson C., The Rat Brain in Stereotaxic Coordinates, 2nd Edition, London: Academic Press, 1986). The upper incisor bar was set at 3.3 mm below the interaural line so that the skull surface between bregma and lambda was horizontal. The coordinates for each probe were identical from bregma except for the lateral measurement as one probe was placed to the left of the midline and the other to the right. Samples were measured from the same side in each rat (e.g. left probe was used to measure ACh and right probe was used to measure DA and 5-HT). Additional burr holes were made for skull screws (stainless steel) and the probes were secured using dental cement.

Following surgery, animals were individually housed in circular chambers (dimensions 450 mm internal diameter, 320 mm wall height) with the microdialysis probes connected to a liquid swivel and a counter-balanced arm to allow unrestricted movement. Rats were allowed a recovery period of at least 16 h with food and water available *ad libitum.* During this time the probes were continuously perfused at a flow rate of 1.2 µl/min with an artificial cerebrospinal fluid (aCSF; Harvard Apparatus, UK) of the following electrolyte composition (in mM): sodium 150; potassium 3.0; magnesium 0.8; calcium 1.4; phosphate 1.0; chloride 155.0. In addition, the aCSF perfusate for the probe measuring ACh levels contained neostigmine (1 µM), a cholinesterase inhibitor which prevented the breakdown of ACh in these samples.

### Microdialysis and Administration of Drugs

The experiment was performed the day after surgery. Dialysate samples were collected every 20 min from 80 min before drug administration until 240 min after drug administration (16 samples in total from each probe; 4 pre-drug and 12 post-drug). The samples were collected into Eppendorf tubes containing 5.0 µl of 0.1 M perchloric acid (samples for measurement of DA and 5-HT only) to prevent oxidation of the neurotransmitters. ACh samples were immediately snap frozen using liquid nitrogen while DA/5-HT samples were stored on dry ice. After the completion of the experiment, all samples were stored at -80°C until analysis. The HPLC analysis of DA and 5-HT in one set of samples and of ACh in the second set was conducted over the remainder of the week following experimentation.

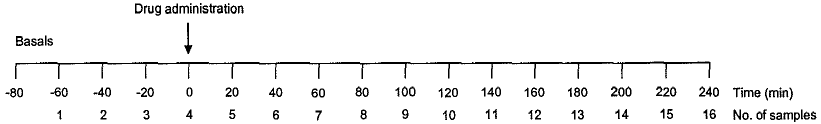

Cariprazine hydrochloride (0.03, 0.1 and 0.3 mg/kg) or vehicle (1% methylcellulose) were administered via the oral route using a gavage (po) using a dosing volume of 2 ml/kg of body weight in combination with escitalopram oxalate (1.0 mg/kg) or vehicle (saline) administered via subcutaneous injection (dosing volume of 1 ml/kg of body weight).

### Dopamine and 5-HT Analysis

Detection and subsequent quantification of both DA and 5-HT in the same dialysis samples involved the use of reverse-phase, ion-pair HPLC coupled with electrochemical detection. Briefly, the method employed a Spherisil reverse-phase column packed with ODS2, 3 µm particles (150 *x* 2.1 mm internal diameter; Capital HPLC, UK). A Gynkotek solvent delivery pump was used to circulate mobile phase (10.5 mM sodium acetate, 0.27 mM EDTA, 2.5 mM 1-octane sulphonic acid, 18% methanol, 0.18% glacial acetic acid, pH 5.5) at a flow rate of 0.2 ml/min and a Jour X-Act in-line degassing unit was used to remove air. Samples (20 µl) were injected onto the column via a Spark Holland Triathlon autosampler with a cooling tray set at 4 °C. An Antec Intro electrochemical detector was used and an Antec VT-03 cell employing a high-density, glassy carbon working electrode (+0.70 V) combined with an Ag/AgCl reference electrode. The electrode signal was integrated using a Turbochrom data acquisition system (Perkin-Elmer). A stock solution of DA and 5-HT (1.0 mM) was prepared by their dissolution in a mixture of equal quantities of deionised water and 0.1 M perchloric acid (in order to prevent oxidation) and stored at 4 °C. A working solution was prepared daily by dilution in aCSF.

### Acetylcholine Analysis

Detection and subsequent quantification of ACh in dialysis samples involved the use of a Unijet microbore ACh/Ch kit (BASi) coupled with electrochemical detection. In this method ACh was separated on the analytical column (1 mm x 530 mm, 5 µm ODS) and converted to hydrogen peroxide (H₂O₂) by the action of acetylcholinesterase (AChE) and choline oxidase (ChOx) which were immobilised on a post column IMER (1 mm x 50 mm, 10 µm AChE-ChOx). The resultant H₂O₂ was reduced on the surface of an enzyme-modified glassy carbon electrode. High concentrations of choline (Ch) can sometimes interfere with the quantitations of ACh but the addition of a precolumn IMER prior to the analytical column, containing immobilized ChOx and catalase (1 mm *x* 50 mm, 10 µm ChOx/catalase) removed any choline present in the sample prior to chromatographic separation. A Gynkotek solvent delivery pump was used to circulate mobile phase (50 mM ortho-phosphoric acid in de-ionised water, 1% ProClin reagent (BASi), pH 8.5) at a flow rate of 0.12 ml/min and a Jour X-Act in-line degassing unit was used to remove air. Samples (20 µl) were injected onto the column via a Spark Holland Triathlon autosampler with a cooling tray set at 4 °C. An Antec Intro electrochemical detector was used and an Antec VT-03 cell employing a high-density, glassy carbon working electrode (-0.1 V) combined with an Ag/AgCl reference electrode. The electrode signal was integrated using a Turbochrom data acquisition system (Perkin-Elmer). A stock solution of ACh (1.0 mM) was prepared by its dissolution in a standard diluent solution and stored at 4 °C. A working solution was prepared daily by dilution in the standard diluent.

### Histology

At the end of the experiments, rats were killed and their brains rapidly removed and stored in a 10% v/v formal saline solution for a minimum of 5 days. Sections (100 µm) were cut on a vibratome and probe placements were visualised and localised with reference to a stereotaxic atlas (Paxinos G, Watson C. The Rat Brain in Stereotaxic Coordinates, 2nd Edition, London: Academic Press, 1986). Data was only reported from animals where probe membranes were correctly positioned in the frontal cortex.

### Drug and Reagents

Cariprazine hydrochloride was stored at room temperature. A factor of 1.09 was used during formulation and all drug doses refer to the free base. A stock solution (0.1635 mg/ml) of the drug was prepared fresh for each experimental day within 30 min of the drug administration by the addition of 1% methylcellulose and gentle agitation under warm water. Additional drug solutions were prepared by a dilution of the stock solution.

Escitalopram oxalate was stored at room temperature. A factor of 1.28 was used during formulation and all drug doses refer to the free base. A solution (1.28 mg/ml) of the drug was prepared fresh for each experimental day within 30 min of the drug administration by the addition of saline and gentle agitation under warm water. The solution was buffered by the dropwise addition of 1M NaOH (pH range 6.8 - 7.6).

All reagents used in HPLC analysis were of HPLC grade. EDTA, methanol, 1-octane sulphonic acid, perchloric acid, glacial acetic acid, sodium acetate, ortho-phosphoric acid, 10% formal saline solution and methylcellulose were obtained from Fisher Scientific (UK). Dopamine hydrochloride and 5-hydroxytryptamine creatinine sulphate were purchased from Sigma Chemical Company (UK) and acetylcholine chloride was purchased from BASi (UK).

### Statistical Analysis

In all experiments, pre- and post-drug data were log transformed and the four pre-drug values were averaged to provide a basal value. Results were back-transformed. Thus, means were adjusted for differences between treatment groups at baseline. Due to occasional chromatography problems not every sample analysed provided data. Therefore, the actual n value varies at each time point. At each post-treatment time point, a one-way analysis of covariance (ANCOVA) was used with (log)baseline as the covariate and treatment as the factor. Each dose of cariprazine hydrochloride with saline was compared to 1% methylcellulose with saline by Williams' test. Each dose of cariprazine hydrochloride with escitalopram oxalate was compared to 1% methylcellulose with escitalopram oxalate by Williams' test. Each dose of cariprazine hydrochloride with escitalopram oxalate was compared to the same dose of cariprazine hydrochloride with saline by the multiple t test. In an initial analysis, studentised residuals were calculated. If the magnitude of the studentised residual was greater than 3, the sample was excluded from the analysis.

Administration of cariprazine hydrochloride alone (0.03, 0.1 and 0.3 mg/kg, po) had no significant effect on the efflux of DA in the frontal cortex. Escitalopram oxalate (1.0 mg/kg, sc) had no effect on basal DA levels.

Cariprazine hydrochloride (0.03 and 0.1 mg/kg, po), in combination with escitalopram oxalate (1.0 mg/kg, sc), had no effect on DA levels versus the corresponding vehicle-treated controls. Cariprazine hydrochloride (0.3 mg/kg, po) + escitalopram oxalate resulted in a small but significant decrease in DA efflux at 80 and 200 min post-drug administration, compared to cariprazine hydrochloride + saline-treated controls (-33% and -40%, respectively).

Administration of cariprazine hydrochloride alone (0.03, 0.1 and 0.3 mg/kg, po) had no significant effect on the efflux of 5-HT in the frontal cortex. Escitalopram oxalate (1.0 mg/kg, sc) had no effect on basal 5-HT levels.Cariprazine hydrochloride (0.03 mg/kg, po) + escitalopram oxalate resulted in a small but significant decrease in 5-HT efflux, 120 min post-drug administration, compared to cariprazine hydrochloride + saline-treated controls (-33%). Cariprazine hydrochloride (0.1 mg/kg, po) + escitalopram oxalate resulted in a significant increase in 5-HT efflux, 60 min post-drug administration, compared to cariprazine hydrochloride + saline-treated controls (106%). Cariprazine hydrochloride (0.3 mg/kg, po) + escitalopram oxalate resulted in small but significant decreases in 5-HT efflux, 200 and 240 min post-drug administration, compared to cariprazine hydrochloride + saline-treated controls (-32% and -48%, respectively).

Administration of cariprazine hydrochloride alone (0.03, 0.1 and 0.3 mg/kg, po) had no significant effect on the efflux of ACh in the frontal cortex. Escitalopram oxalate (1.0 mg/kg, sc) resulted in significant decreases in basal ACh levels, 100 and 240 min post-administration, compared to vehicle + saline-treated controls (-55% and -56%, respectively).

Cariprazine hydrochloride (0.03, 0.1 and 0.3 mg/kg, po), in combination with escitalopram oxalate (1.0 mg/kg, sc), had no effect on ACh levels versus the corresponding vehicle-treated controls.

Cariprazine hydrochloride, at 0.03, 0.1 and 0.3 mg/kg po, had no effect on extracellular levels of DA, 5-HT and ACh in the rat frontal cortex. Escitalopram oxalate (1.0 mg/kg sc) had little or no effect on these neurotransmitters with the exception of a small decrease in ACh levels at two time points.

Cariprazine hydrochloride and escitalopram, administered in combination, also had little or no effect on extracellular levels of DA, 5-HT and ACh in the rat frontal cortex.

### Example 2: Cariprazine Hydrochloride in a Chronic Mild Stress Model of Depression

This study evaluated the antidepressant effect of cariprazine hydrochloride in the chronic mild stress (CMS) model of depression. A comparison to the efficacy of the tricyclclic antidepressant compound imipramine was also made.

Male Wistar rats were adapted to laboratory and housing conditions for 3 weeks, followed by adoption to consumption of 1% sucrose solution for an additional 5 weeks before being separated into control and to-be-stressed groups.

Chronic stress was applied to the allocated group for seven weeks. The following stressors were used: food and/or water deprivation, cage tilting (45 degrees backwards), intermittent illumination, paired housing, soiled cage (250 mL of tap water in sawdust bedding), stroboscopic illumination (150 flashed per minute), no stress. All stressors were 10-14 hours in duration and were applied individually and continuously, once or twice weekly, during the day and at night.

Consumption of sucrose solution was measured in 1-hour tests conducted on all animals (i.e., controls and stressed) once weekly throughout the entire experiment. The tests were preceded by 14 hours of food and water deprivation. On the basis of their sucrose intakes following the initial 2 weeks of stress, both stressed and control animals received once-daily chronic (5 weeks) IP injections of vehicle (1% methyl cellulose, 1 mL/kg), cariprazine hydrochloride (0.0625, 0.25 or 1.0 mg/kg), or imipramine (10 mg/kg) as reference treatment. The drugs were administered at approximately 10.00 am and the weekly sucrose tests were carried out 24 hours following the last drug injection. Stress was continued throughout the entire period of treatment. Typically 2-3 animals out of 8 did not respond to drug treatment in each group. These animals were considered weak responders/non-responders and were eliminated from the analysis.

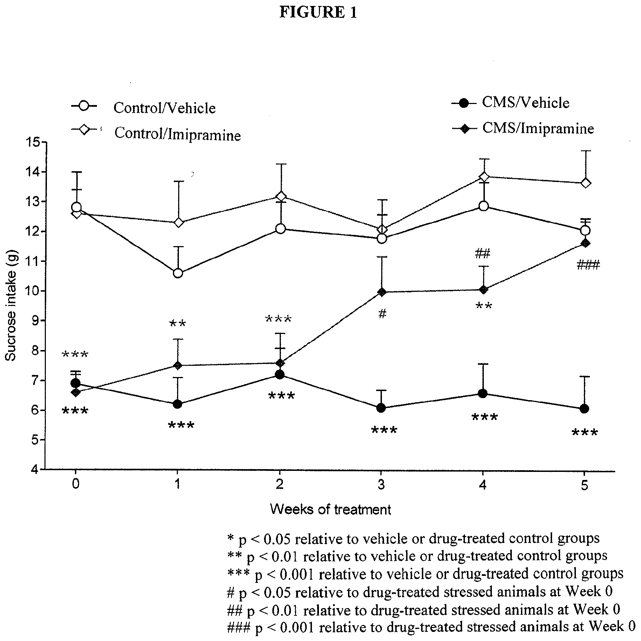

The effects of chronic treatment with vehicle (1% methylcellulose, 1mL/kg, IP, once daily) and imipramine (10 mg/kg, IP, once daily) on the consumption of 1% sucrose solution in controls (open symbols) and in animals exposed to CMS (closed symbols) in shown in Figure 1.

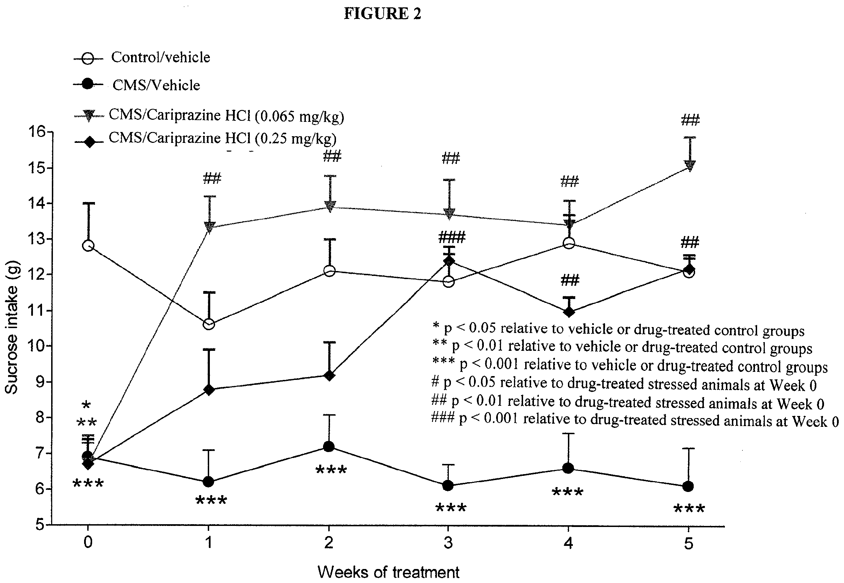

The effects of chronic treatment with vehicle (1% methylcellulose, 1mL/kg, IP, once daily) and cariprazine hydrochloride (0.065 and 0.25 mg/kg, IP, once daily) on the consumption of 1% sucrose solution in controls (open symbols) and in animals exposed to CMS (closed symbols) in shown in Figure 2,

CMS caused a substantial decrease in the consumption of 1% sucrose solution. This decrease was fully reversed by chronic treatment with imipramine.

Cariprazine hydrochloride administered at doses of 0.065 and 0.25 mg/kg dose-dependently increased sucrose drinking in stressed animals without any significant effect in controls (non-stressed). The magnitude of effect of the most active dose of 0.065 mg/kg was greater than that of imipramine. Also, the onset of action of this dose of cariprazine hydrochloride was faster, i.e., increases in sucrose drinking were evident already at Week 1, whereas the effect of imipramine was evident at Week 3.

The highest dose of cariprazine hydrochloride tested, 1 mg/kg, was inactive in the CMS model. At this dose, cariprazine hydrochloride did not change sucrose intake in stressed animals and significantly decreased the sucrose consumption in control animals.The 1 mg/kg dose is a strongly sedative dose at which the D₂ antagonist nature of the compound may predominate resulting in a decrease of sucrose intake even in the non-stressed animals.

### Example 3: Cariprazine Hydrochloride and Escitalopram Oxalate in a Chronic Mild Stress Model of Depression

Similar to the study described in Example 2, additional studies were undertaken to further evaluate the antidepressant effect of cariprazine hydrochloride, administered alone, or in combination with escitalopram oxalate, in a chronic mild stress model of depression

In the chronic mild stress (CMS) model, rats subjected to a variety of mild stressors for a prolonged period of time show a substantial decrease in their responsiveness to rewarding stimuli. This deficit is usually monitored by a decrease in the consumption of a 1% sucrose solution, but can also be seen in other tests, such as place preference conditioning or intracranial self-stimulation. The subsensitivity to reward appears to reflect anhedonia (inability to experience pleasure), which is a core symptom of major depressive disorders. Clinically approved antidepressants have shown activity in this animal model by reversing the effects of CMS on sucrose consumption. See, e.g., Sanchez C, Gruca P, Papp M, Behavioural Pharmacology 14, 465-470, 2003.

### Animals

Male Wistar rats (Charles River, Germany) were brought into the laboratory two months before the start of the experiment. Except as described below, the animals were singly housed with food and water freely available, and were maintained on a 12-h light/dark and in a constant temperature (22 ± 2°C) and humidity (50 ± 5%) conditions.

### Stress Procedure

The animals were first trained to consume a 1% sucrose solution; training consisted of nine 1h baseline tests in which sucrose was presented, in the home cage, following 14h food and water deprivation. The sucrose intake was measured by weighing pre-weighed bottles containing the sucrose solution, at the end of the test. Subsequently, sucrose consumption was monitored, under similar conditions, at weekly intervals throughout the whole experiment.

On the basis of their sucrose intakes in the final baseline test, the animals were divided into two matched groups. One group of animals was subjected to the chronic mild stress procedure for a period of 7 consecutive weeks. Each week of stress regime consisted of: two periods of food or water deprivation, two periods of 45 degree cage tilt, two periods of intermittent illumination (lights on and off every 2h), two periods of soiled cage (250 ml water in sawdust bedding), one period of paired housing, two periods of low intensity stroboscopic illumination (150 flashes/min), and three periods of no stress. All stressors were 10 - 14 h of duration and were applied individually and continuously, day and night. Control animals were housed in separate rooms and had no contact with the stressed animals. They were deprived of food and water for 14h preceding each sucrose test, but otherwise food and water were freely available in the home cage.

### Drug Administration

On the basis of their sucrose intake scores following initial 2 weeks of stress, the stressed animals were further divided into matched subgroups (n = 8 per group) and for subsequent five weeks they received once daily intraperitoneal injections of vehicle (1% methylcellulose, 1 ml/kg), cariprazine hydrochloride (0.01, 0.03 and 0.065 mg/kg) alone, escitalopram oxalate (2 mg/kg) alone or cariprazine hydrochloride (0.01 and 0.03 mg/kg) in combination with escitalopram oxalate (2 mg/kg). One group of control, non-stressed animals (n = 8) received similar administration of vehicle (1% methylcellulose, 1 ml/kg). The drugs were administered at approx. 10.00 and the weekly sucrose tests were carried out 24h following the last drug injections. Stress was continued throughout the entire period of treatment. The doses of cariprazine hydrochloride and escitalopram oxalate are expressed in mg free base per kg body weight.

### Statistics

All results obtained in this study were analyzed by multiple analyses of variance with three between-subjects factors (stress/control, drug treatments and successive sucrose tests). The Fisher's LSD test was used for post-hoc comparisons of means.

Chronic mild stress caused a gradual decrease in the consumption of 1% sucrose solution. In the final baseline test, all animals drank approximately 13 g of sucrose solution. Following initial two weeks of stress, intakes remained at similar level in controls but fell to 7.5 g in stressed animals, resulting in a significant Group effect [F(1,62) = 92.179; p<0.001]. Such a difference between control and stressed animals treated with vehicle persisted at similar level for the remainder of the experiment.

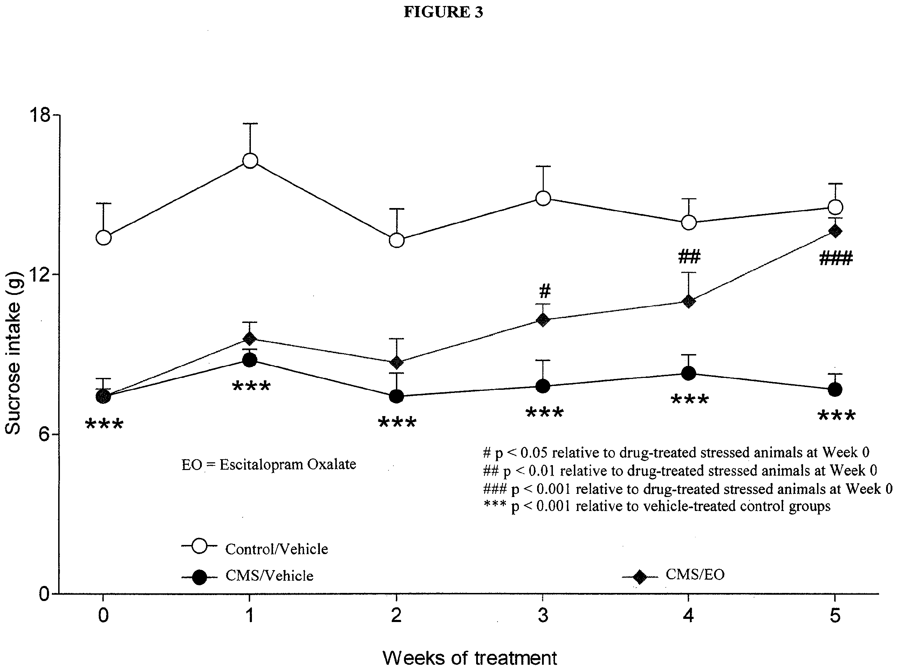

Figure 3 shows the effects of chronic treatment with vehicle (1% methylcellulose, 1 ml/kg, IP, once daily) and escitalopram oxalate (2 mg/kg. IP, once daily) on the consumption of 1% sucrose solution in controls (open symbols) and in animals exposed to chronic mild stress (closed symbols). Treatment commenced following 2 weeks of stress. Values are means +/- SEM (standard error of the mean).

As compared to vehicle administration, escitalopram oxalate gradually increased the sucrose consumption in all stressed animals, resulting in a significant Treatment effect [F(1,84) = 27.447; p<0.001] and Treatment x Weeks interaction [F(5,84) = 4.135; p=0.002].

As compared to Week 0 scores, the increases in sucrose intake in stressed animals administered escitalopram oxalate reached statistical significance after three weeks of treatment (p=0.024) and this effect was enhanced thereafter.

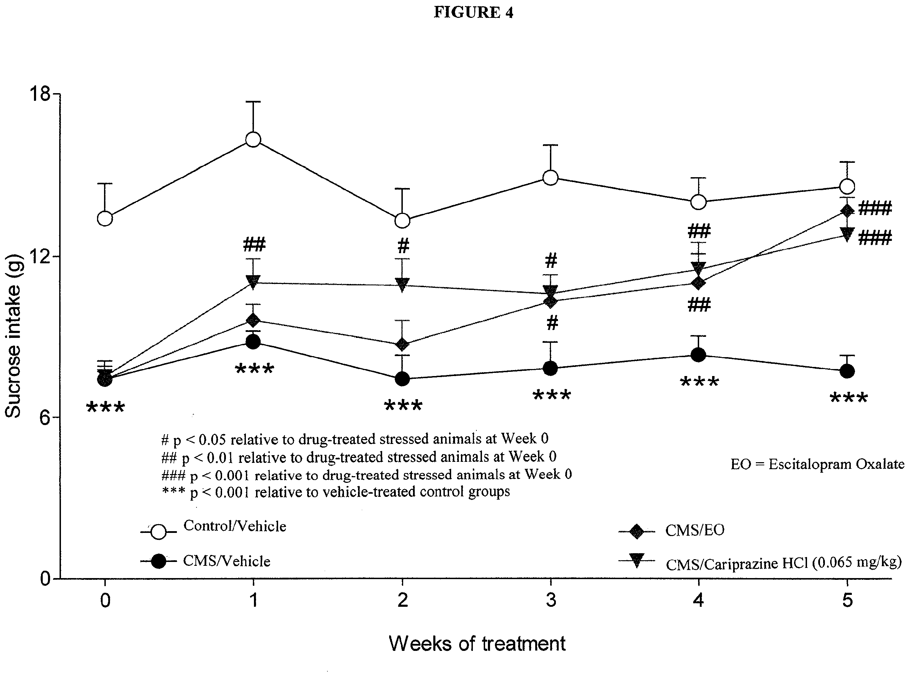

Figure 4 shows the effects of chronic treatment with vehicle, escitalopram oxalate (2 mg/kg. IP, once daily) and cariprazine hydrochloride (0.065 mg/kg base, IP, once daily) on the consumption of 1% sucrose solution in controls (open symbols) and in animals exposed to chronic mild stress (closed symbols). Treatment commenced following 2 weeks of initial stress. Values are means +/- SEM.

As compared to vehicle administration, cariprazine hydrochloride gradually increased the sucrose consumption in stressed animals, resulting in a significant Treatment effect [F(1,84) = 37.782; p<0.001] but not Treatment x Weeks interaction [F(5,84) = 2.057; p=0.0784].

As compared to Week 0 scores, the increases in sucrose intake in stressed animals administered cariprazine hydrochloride reached statistical significance after the first week of treatment (p=0.009) and this effect was maintained and enhanced thereafter. One stressed animal showed rather weak response to 0.065 mg/kg of cariprazine hydrochloride but it was not eliminated from the analysis.

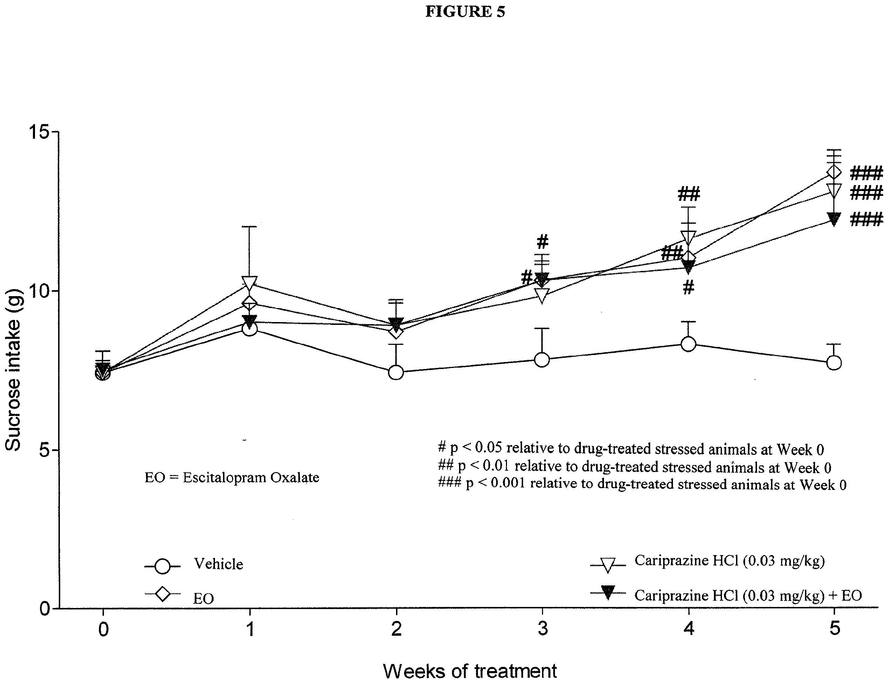

Figure 5 shows the effects of chronic treatment with vehicle, escitalopram oxalate (2 mg/kg. IP, once daily) and cariprazine hydrochloride (0.03 mg/kg base, IP, once daily) administered alone or in combination with escitalopram oxalate on the consumption of 1% sucrose solution in animals exposed to chronic mild stress. Treatment commenced following 2 weeks of initial stress. Values are means +/- SEM.

As compared to vehicle administration, cariprazine hydrochloride alone gradually increased the sucrose consumption in stressed animals, resulting in a significant Treatment effect [F(1,84) = 16.437; p<0.001] but not Treatment x Weeks interaction [F(5,84) = 1.819; NS].

As compared to Week 0 scores, the increases in sucrose intake in stressed animals administered cariprazine hydrochloride reached statistical significance after four weeks of treatment (p=0.003) and this effect was enhanced thereafter. Two stressed animals showed rather weak response to 0.03 mg/kg of cariprazine hydrochloride treatment but they were not eliminated from the analysis.

As compared to vehicle administration, cariprazine hydrochloride administered in combination with escitalopram oxalate produced similar effects, i.e. gradual increase of the sucrose consumption in stressed animals, resulting in a significant Treatment effect [F(1,84) = 12.595; p<0.001] but not Treatment x Weeks interaction [F(5,84) = 1.639; NS].

As compared to Week 0 scores, the increases in sucrose intake in stressed animals administered cariprazine hydrochloride reached statistical significance after three weeks of treatment (p=0.032) and this effect was enhanced thereafter. Three stressed animals showed weak or lack of response to joint injections of cariprazine hydrochloride (0.03 mg/kg) and escitalopram oxalate but they were was not eliminated from the analysis.

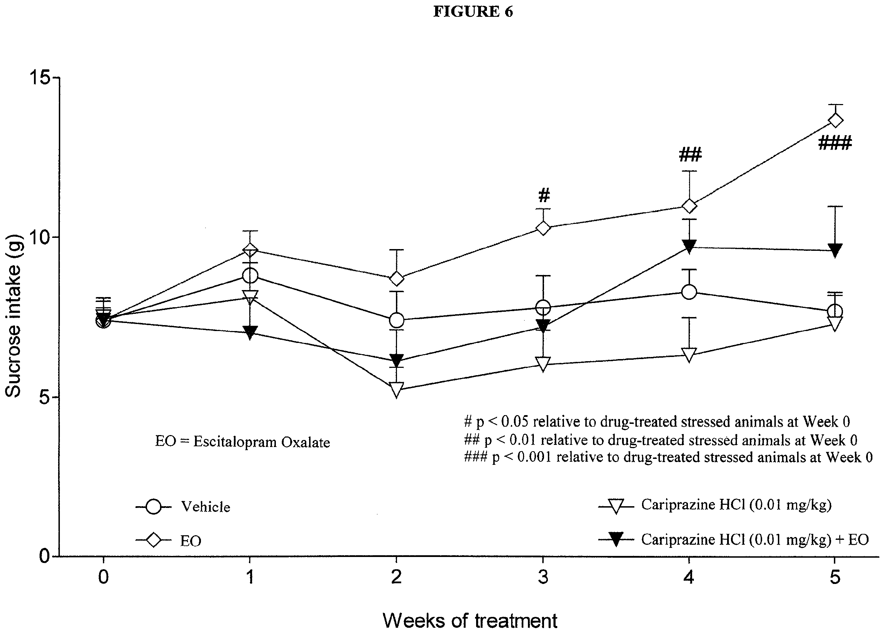

Figure 6 shows the effects of chronic treatment with vehicle, escitalopram oxalate (2 mg/kg. IP, once daily) and cariprazine hydrochloride (0.01 mg/kg base, IP, once daily) administered alone or in combination with escitalopram oxalate on the consumption of 1% sucrose solution in animals exposed to chronic mild stress. Treatment commenced following 2 weeks of initial stress. Values are means +/- SEM.

As compared to vehicle administration, cariprazine hydrochloride significantly decreased the sucrose consumption when administered alone [Treatment effect: F(1,84) = 5.035; p=0.027, Treatment x Weeks interaction [F(5,84) = 0.561; NS] but this effect never reached statistical significance when compared to Week 0 scores.

When administered in combination with escitalopram oxalate, cariprazine hydrochloride decreased the sucrose consumption during the first two weeks of treatment and increased the intakes in the last two weeks, resulting in a non-significant Treatment effect [F(1,84) = 0.12; NS] and Treatment x Weeks interaction [F(5,84) = 1.562; NS]. At the end of treatment period three animals receiving 0.1 mg/kg of cariprazine hydrochloride plus escitalopram oxalate showed no enhancement of sucrose consumption over the Week 0 values.

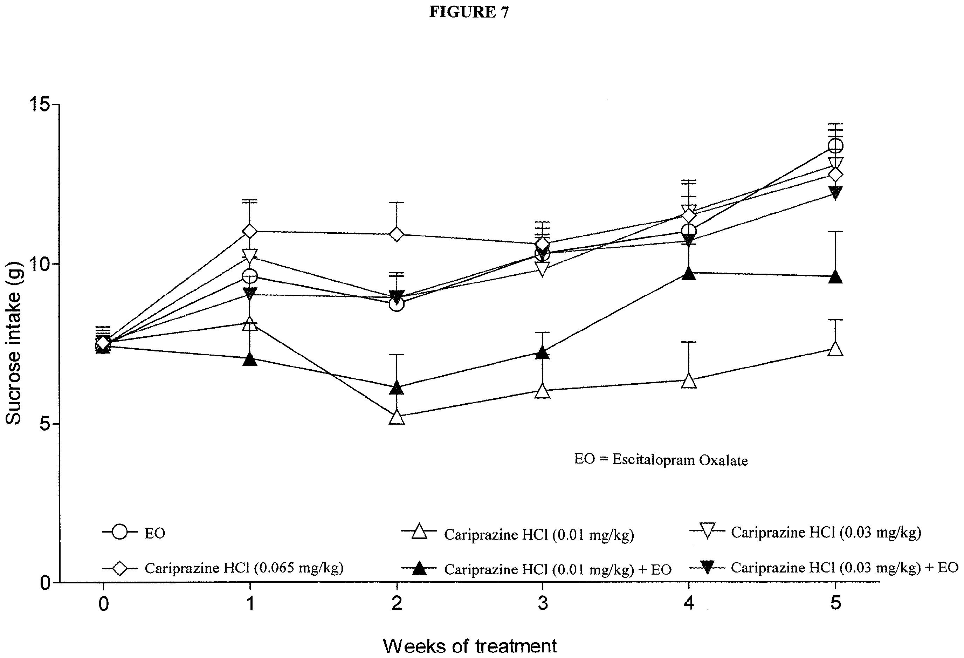

Figure 7 shows the effects of chronic treatment with cariprazine hydrochloride (0.01, 0.03 and 0.065 mg/kg) alone, escitalopram oxalate (2 mg/kg) alone or cariprazine hydrochloride (0.01 and 0.03 mg/kg) in combination with escitalopram oxalate on the consumption of 1% sucrose solution in animals exposed to chronic mild stress. Treatment commenced following 2 weeks of initial stress. Values are means +/- SEM.

Before the stress procedure was initiated (baseline) the to-be-stressed animals were slightly smaller than the controls (418 and 433 g, respectively) and after initial two weeks of stress (Week 0) this difference was enhanced, resulting in a significant Group effect [F(1,62) = 8.310; p=0.005].

As compared to the vehicle-treated group, neither escitalopram oxalate [F(1,14) = 0.157; NS] nor cariprazine hydrochloride alone [0.065 mg/kg: F(1,14) = 2.111; NS, 0.03 mg/kg: F(1,14) = 0.406; NS, 0.01 mg/kg: F(1,14) = 1.229; NS] or in combination with escitalopram oxalate [0.03 mg/kg: F(1,14) = 0.308; NS, 0.01mg: F(1,14) = 0.022; NS] affected the body weights of stressed animals.

The results of this study show that the CMS procedure causes a substantial decrease in the consumption of 1% sucrose solution, and that this deficit can be fully reversed by chronic treatment with escitalopram oxalate (three weeks to first significant effects) The effect of imipramine, which was tested as reference treatment in parallel studies, also reached significance after three weeks of administration.

Cariprazine hydrochloride appears to be active in the CMS model of depression; when administered alone at doses of 0.065 and 0.03 mg/kg, the compound gradually increased the sucrose drinking in most of the stressed animals.

The magnitude of action of cariprazine hydrochloride was comparable to that of escitalopram oxalate (full recovery at the end of treatment period) but the onset of action of the most active dose of 0.065 mg/kg was substantially faster; the first significant effect was observed already following the first week of administration (compared to three weeks required by the dose of 0.03 mg/kg cariprazine hydrochloride and by escitalopram oxalate) and this effect was enhanced thereafter.

Cariprazine hydrochloride, administered in combination with escitalopram oxalate, caused similar effects (both in terms of the magnitude and the onset of action) to those observed following single injections of both compounds.

At the lowest dose of 0.01 mg/kg, cariprazine hydrochloride decreased the sucrose consumption when administered alone and escitalopram oxalate reversed this decrease in the last two weeks of treatment.

### Reference Example 4A: Evaluation of Cariprazine Hydrochloride and Escitaloprann Oxalate for Anti-Manic Effects in the Mouse Amphetamine-Chlordiazepoxide Hyperactivity Model

Cariprazine hydrochloride and escitalopram oxalate were evaluated for anti-manic effects using the amphetamine/chlordiazepoxide (AMPH/CDP) hyperactivity test.

### Animals

Male C57B1/6J mice from Jackson Laboratories (Bar Harbor, Maine) were used in this study. Mice were received at 6-weeks of age. Upon receipt, mice were assigned unique identification numbers (tail marked) and were group housed with 4 mice/cage. All animals remained housed in groups of four during the remainder of the study. All mice were acclimated to the colony room for at least two weeks prior to testing and were subsequently tested at an average age of 8 weeks of age. During the period of acclimation, mice were examined on a regular basis, handled, and weighed to assure adequate health and suitability. Mice were maintained on a 12/12 light/dark cycle. The room temperature was maintained between 20 and 23°C with a relative humidity maintained between 30% and 70%. Chow and water were provided *ad libitum* for the duration of the study. In each test, animals were randomly assigned across treatment groups.

### Drug Administration

The following compounds were used:
- d-Amphetamine sulfate (*Sigma,* 4.0 mg/kg) and chlordiazepoxide (CDP; *Sigma*, 2.5 mg/kg) were dissolved in sterile water and were administered intraperitoneally at a dose volume of 10 ml/kg.
- Valproate (VPA; *Sigma, Lot 064K1585,* 400 mg/kg) was dissolved in sterile water and was administered intraperitoneally at a dose volume of 10 ml/kg 30 min prior to water or d-amphetamine/CDP mixture.
- Cariprazine hydrochloride (0.03, 0.10, and 0.30 mg/kg) and escitalopram oxalate (0.5, 2.0, and 5.0 mg/kg) were dissolved in sterile water and administered orally at a dose volume of 10 ml/kg 60 min prior to water or d-amphetamine/CDP mixture. The doses of cariprazine hydrochloride and escitalopram oxalate are expressed in mg free base per kg body weight.

### Methods

The open field test (OF) is used to assess both anxiety-like behavior and motor activity. The open field chambers are plexiglas square chambers (27.3 x 27.3 x 20.3 cm; Med Associates Inc., St Albans, VT) surrounded by infrared photobeam sources (16 x 16 x 16). Distance traveled is measured by consecutive beam breaks. Total distance traveled during the test session was used as an index of activity. After 60 minute pretreatment with water, cariprazine hydrochloride or escitalopram oxalate, or 30 minute pretreatment with valproate, mice were injected with water or d-amphetamine/CDP mixture ('mixture') and placed in the OF chambers for a 60 min test session. At the end of each open field test session the OF chambers were thoroughly cleaned.

### Statistical Analysis

Data was analyzed by analysis of variance (ANOVA) followed by Fisher PLSD post-hoc analysis when appropriate. An effect was considered significant ifp < 0.05. Outliers that fell above and below two standard deviations from the mean were removed from the final analysis.

Mice treated with cariprazine hydrochloride or escitalopram oxalate showed no signs of toxicity or sedation during pretreatment or testing.

### Total Distance Traveled

The summary of the effects (i.e., the total distance traveled summed over the 60 minute test period) produced by different treatment regimens in the amphetamine-chlordiazepoxide mouse model of mania are shown in Tables 1-4.

**TABLE 1: Effect of the Administration of Cariprazine Hydrochloride or Escitalopram Oxalate on the Basal Locomotor Activity of Mice**

| Test Condition | Drug Treatment | Total Distance Traveled (cm/60 min) [mean ± SEM] | n |
|---|---|---|---|
| Water | Vehicle | 5103 ± 679 | 12 |
| | Valproate (400 mg/kg) | 4471 ± 298 | 10 |
| | Cariprazine HCl (0.03 mg/kg) | 4056 ± 427 | 12 |
| | Cariprazine HCl (0.10 mg/kg) | 1726 ± 112 | 11 |
| | Cariprazine HCl (0.30 mg/kg) | 917 ± 73^{#} | 12 |
| | Escitalopram oxalate (0.50 mg/kg) | 4435 ± 389 | 11 |
| | Escitalopram oxalate (2.0 mg/kg) | 5939 ± 369 | 11 |
| | Escitalopram oxalate (5.0 mg/kg) | 6499 ± 445 | 11 |

| | | | |
|---|---|---|---|
| *^{#} significant difference from vehicle-treated controls (p<0.05)* | | | |

Neither valproate nor escitalopram oxalate (0.5, 2.0, and 5.0 mg/kg) had a significant effect on basal activity compared to water-treated mice. Cariprazine hydrochloride (0.30 mg/kg) significantly decreased the basal activity of the mice compared to water (p = 0.032), and 0.10 mg/kg cariprazine hydrochloride exhibited a trend toward a significant decrease in basal locomotor activity compared to water (p = 0.090).

**TABLE 2**

| Test Condition | Drug Treatment | Total Distance Traveled (cm/60 min) [mean ± SEM] | n |
|---|---|---|---|
| water | Vehicle | 5103 ± 679 | 12 |
| AMPH/CDP | Vehicle | 33591 ± 1106^{#} | 11 |

| | | | |
|---|---|---|---|
| ^{#} *significant difference from water controls (p<0.05)* | | | |

Administration of the d-amphetamine/chlordiazepoxide mixture (AMPH/CDP) resulted in a marked increase in locomotor activity compared to water.

**TABLE 3**

| Test Condition | Drug Treatment | Total Distance Traveled (cm/60 min) [mean ± SEM]] | n |
|---|---|---|---|
| AMPH/CDP | Vehicle | 33591 ± 1106 | 11 |
| AMPH/CDP | Valproate (400 mg/kg) | 17496 ± 1788^{*} | 12 |

| | | | |
|---|---|---|---|
| *^{*} significant difference from the vehicle-treated group (p<0.05)* | | | |

Administration of valproate significantly decreased the d-amphetamine/chlordiazepoxide mixture (AMPH/CDP)-induced hyperactivity in mice

**TABLE 4: Effect of the Administration of Cariprazine Hydrochloride and Escitalopram Oxalate on the d-Amphetamine/Chlordiazepoxide Mixture (AMPH/CDP)-Induced Hyperactivity in Mice**

| Test Condition | Drug Treatment | Total Distance Traveled (cm/60 min) [mean ± SEM]] | n |
|---|---|---|---|
| AMPH/CDP | Vehicle | 33591 ± 1106 | 11 |
| | Cariprazine HCl (0.03 mg/kg) | 32122 ± 2942 | 12 |
| | Cariprazine HCl (0.10 mg/kg) | 13688 ± 2349^{*} | 12 |
| | Cariprazine HCl (0.30 mg/kg) | 2525 ± 204^{*} | 11 |
| | Escitalopram oxalate | 30067 ± 1901 | 12 |
| | (0.50 mg/kg) | | |
| | Escitalopram oxalate (2.0 mg/kg) | 36112 ± 11934 | 11 |
| | Escitalopram oxalate (5.0 mg/kg) | 39639 ± 1582^{*} | 11 |

| | | | |
|---|---|---|---|
| *^{*}significant difference from the vehicle-treated group (p<0.05)* | | | |

Valproate (400 mg/kg) and cariprazine hydrochloride (0.10 and 0.30 mg/kg) significantly decreased the mixture-induced hyperactivity compared to water (p < 0.0001). Escitalopram oxalate (5.0 mg/kg) increased mixture-induced hyperactivity compared to water (p = 0.003). Escitalopram oxalate (0.5 mg/kg) exhibited a trend toward a significant decrease in mixture-induced hyperactivity compared to water (p = 0.077).

### Reference Example 4B: Evaluation of Cariprazine Hydrochloride and Escitalopram Oxalate for Anti-Manic Effects in the Mouse Amphetamine-Chlordiazepoxide Hyperactivity Model

Cariprazine hydrochloride and escitalopram oxalate were evaluated for anti-manic effects using the amphetamine/chlordiazepoxide (AMPH/CDP) hyperactivity test.

### Animals

Male C57B1/6J mice (8 weeks old) from Jackson Laboratories (Bar Harbor, Maine) were used in this study. Upon receipt, mice were assigned unique identification numbers (tail marked) and were group housed (4 mice/cage) in OPTI mouse ventilated cages. All animals remained housed in groups of four during the remainder of the study. During the period of acclimation, mice were examined on a regular basis, handled, and weighed to assure adequate health and suitability. Mice were maintained on a 12/12 light/dark cycle. The room temperature was maintained between 20 and 23°C with a relative humidity maintained between 30% and 70%. Chow and water were provided *ad libitum* for the duration of the study. In each test, animals were randomly assigned across treatment groups.

### Drug Administration

The following compounds were used.
- d-Amphetamine sulfate (*Sigma,* 4.0 mg/kg) and chlordiazepoxide (CDP; *Sigma*, 2.5 mg/kg) were dissolved in sterile water and were administered intraperitoneally at a dose volume of 10 ml/kg.
- Valproate (VPA; *Sigma,* 400 mg/kg) was dissolved in sterile water and was administered intraperitoneally at a dose volume of 10 ml/kg 30 min prior to water or d-amphetamine/CDP mixture.
- Cariprazine hydrochloride (0.03 and 0.10 mg/kg) and escitalopram oxalate (0.10 and 0.50 mg/kg) were dissolved in sterile water and administered orally at a dose volume of 10 ml/kg 60 min prior to water or d-amphetamine/CDP mixture. The doses of cariprazine hydrochloride and escitalopram oxalate are expressed in mg free base per kg body weight.

### Methods

The open field test (OF) was used to assess both anxiety-like behavior and motor activity. The open field chambers are plexiglas square chambers (27.3 x 27.3 x 20.3 cm; Med Associates Inc., St Albans, VT) surrounded by infrared photobeam sources (16 x 16 x 16). Distance traveled is measured by consecutive beam breaks. Total distance traveled during the test session was used as an index of activity. Following pretreatment with water, valproate or test compounds (cariprazine hydrochloride, escitalopram oxalate), mice were injected with either water or d-amphetamine/CDP mixture ('mixture') and placed in the OF chambers for a 60 min test session. At the end of each open field test session the OF chambers were thoroughly cleaned.

### Statistical Analysis

Data was analyzed by analysis of variance (ANOVA) followed by Fisher PLSD post-hoc analysis when appropriate. An effect was considered significant if p < 0.05. Outliers that fell above and below two standard deviations from the mean were removed from the final analysis.

None of the compounds tested produced any clinical signs in the mice during pretreatment or testing.

### Total Distance Traveled

The summary of the effects (i.e., the total distance traveled summed over the 60 minute test period) produced by different treatment regimens in the amphetamine-chlordiazepoxide (AMPH/CDP) mouse model of mania are shown in Tables 5-8.

**TABLE 5**

| Test Condition | Drug Treatment | Total Distance Traveled (cm/60 min) [Mean ± SEM] | n |
|---|---|---|---|
| Water | Vehicle | 4316 ± 631 | 12 |
| | Valproate (400 mg/kg) | 3365 ± 612 | 12 |
| | Cariprazine HCl (0.03 mg/kg) | 4013 ± 343 | 11 |
| | Cariprazine HCl (0.10 mg/kg) | 2697 ± 259 | 11 |
| | Escitalopram oxalate (0.10 mg/kg) | 5551 ± 382 | 12 |
| | Escitalopram oxalate (0.50 mg/kg) | 5238 ± 242 | 12 |
| | Cariprazine HCl (0.03 mg/kg) + Escitalopram oxalate (0.10 mg/kg) | 4160 ± 296 | 12 |
| | Cariprazine HCl (0.03 mg/kg) + Escitalopram oxalate (0.50 mg/kg) | 3830 ± 410 | 11 |
| | Cariprazine HCl (0.10 mg/kg) + Escitalopram oxalate (0.10 mg/kg) | 2771 ± 343 | 11 |
| | Cariprazine HCl (0.10 mg/kg) + Escitalopram oxalate (0.50 mg/kg) | 1972 ± 309 | 12 |

Compared to water, the drug treatments shown in Table 5 did not produce a significant effect on basal locomotor activity of mice.

**TABLE 6**

| Test Condition | Drug Treatment | Total Distance Traveled (cm/60 min) [Mean ± SEM] | n |
|---|---|---|---|
| Water | Vehicle | 4316 ± 631 | 12 |
| AMPH/CDP | Vehicle | 36086 ± 2978^{#} | 12 |

### ^{#} significant difference from water controls (p<0.05)

Mice treated with the mixture (CDP+amphetamine) showed a significantly higher locomotor activity compared to water-treated mice.

**TABLE 7**

| Test Condition | Drug Treatment | Total Distance Traveled (cm/60 min) [Mean ± SEM] | n |
|---|---|---|---|
| AMPH/CDP | Vehicle | 36086 ± 2978 | 12 |
| AMPH/CDP | Valproate (400 mg/kg) | 18478 ± 2961^{*} | 12 |

### ^{*}significant difference from the vehicle-treated group (p<0.05)

Administration of valproate (400 mg/kg) significantly decreased the d-amphetamine/chlordiazepoxide mixture (AMPH/CDP)-induced hyperactivity in mice.

**TABLE 8: Effect of the Administration of Cariprazine Hydrochloride and Escitalopram Oxalate, Alone or in Combination, on the d-Amphetamine/Chlordiazepoxide Mixture (AMPH/CDP)-Induced Hyperactivity in Mice**

| Test Condition | Drug Treatment | Total Distance Traveled (cm/60 min) [Mean ± SEM] | n |
|---|---|---|---|
| AMPH/CDP | Vehicle | 36086 ± 2978 | 12 |
| | Cariprazine HCl (0.03 mg/kg) | 32628 ± 2510 | 11 |
| | Cariprazine HCl (0.10 mg/kg) | 22594 ± 2838^{*} | 12 |
| | Escitalopram oxalate (0.10 mg/kg) | 39700 ± 3477 | 12 |
| | Escitalopram oxalate (0.50 mg/kg) | 40881 ± 3123 | 12 |
| | Cariprazine HCl (0.03 mg/kg) + Escitalopram oxalate (0.10 mg/kg) | 30350 ± 1313^{*, &} | 12 |
| | Cariprazine HCl (0.03 mg/kg) + Escitalopram oxalate (0.50 mg/kg) | 33343 ± 2060% | 12 |
| | Cariprazine HCl (0.10 mg/kg) + Escitalopram oxalate (0.10 mg/kg) | 16191 ± 2101^{*,∼,&} | 11 |
| | Cariprazine HCl (0.10 mg/kg) + Escitalopram oxalate (0.50 mg/kg) | 10730 ± 790^{*,∼,%} | 11 |

| | | | |
|---|---|---|---|
| *^{*} significant difference from the vehicle-treated group (p<0.05)* ^{∼} *significant difference from the cariprazine HCl (0.10 mg*/*kg) treated group (p<0.05)* *^{&} significant difference from the escitalopram (0.10 mg*/*kg) treated group (p<0.05)* *^{%} significant difference from the escitalopram (0.50 mglkg) treated group (p<0.05)* | | | |

The enhanced locomotor activity produced by the CDP+amphetamine mixture was significantly attenuated by valproate and cariprazine hydrochloride (0.10 mg/kg). Escitalopram oxalate alone, at either dose tested, had no significant effect on mixture-induced locomotor activity. Combinations of cariprazine hydrochloride (0.10 mg/kg) and escitalopram oxalate (0.10 and 0.50 mg/kg) significantly decreased the mixture-induced hyperactivity. In addition, the decrease produced by the combinations was significantly larger in magnitude than that produced by cariprazine hydrochloride (0.10 mg/kg) alone. Combination of the low dose of cariprazine hydrochloride (0.03 mg/kg) and escitalopram oxalate (0.10 mg/kg) also produced a significant decrease in mixture-induced locomotor activity.

### Reference Example 5: The Effect of a Combination of Cariprazone Hydrochloride and Escitalopram Oxalate on the Mouse Brain Glycogen Synthase Kinase-3 Activity

Glycogen synthase kinase-3 (GSK) is as an important enzyme that modulates neuronal function. Abnormal GSK3 activity has been implicated in mood disorders and schizophrenia.

The aim of this study is to evaluate combinations of cariprazine hydrochloride and escitalopram oxalate for the ability to stimulate phosphorylation of GSK3 in mouse brain.

### Animals and Environment

Experiments will be carried out in adult male C57BL/6 obtained from Jackson Laboratories (Bar Harbor, Maine). Before experiments, animals will be housed four or five to a cage at 23°C on a 12 h light/12 h dark cycle with *ad libitum* access to food and water.

### Drug Administration

LiCl (200 mg/kg) will be used as the reference compound and vehicle-injected animals will be used as control. Lithium will be dissolved in sterile injectable water and administered IP at a dose volume of 10 ml/kg 30 min prior to sacrificing the animal. Cariprazine hydrochloride, dissolved in sterile injectable water, will be administered orally (gavage) at a dose of 0.2 mg/kg (dose volume of 10 ml/kg) 60 minutes prior to sacrificing the animals. Doses are expressed as mg free base/kg body weight. Escitalopram oxalate, dissolved in sterile injectable water, will be administered subcutaneously (SC) at a dose of 1 mg/kg (dose volume of 10 ml/kg) 60 minutes prior to sacrificing the animals. Doses are expressed as mg free base/kg body weight. The effect of the combination will be evaluated following the co-administration of cariprazine hydrochloride (0.2 mg/kg PO) and escitalopram oxalate (1 mg/kg SC) 60 minutes prior to sacrificing animals.

### Antibodies

The anti-phospho-GSK-3*α*/*β* Ser-21/9 may be purchased from Cell Signaling Technology (Beverly, MA). The anti-DARPP-32 may be obtained from BD Transduction Laboratories (Lexington, KY).

### GSK-3 Activity Assay

For each determination of kinase activity, various regions of mouse brain will be rapidly dissected out on an ice-cold surface and homogenized at 4°C in lysis buffer (20 mM Tris, pH 8.0/137 mM NaCl/10% glycerol/1% Nonidet P-40/0.5 mM sodium orthovanadate/2 *µ*M okadaic acid and a mixture of protease inhibitors (Sigma-Aldrich). GSK-3*α* and -3*β* will be immunoprecipitated at 4°C from 1 mg of protein extract with 2 *µ*g of anti-GSK-3*α*/*β* monoclonal antibody and protein-A Sepharose (Amersham Pharmacia Bioscience). Immunoprecipitates will then be incubated in assay buffer (20 mM Tris, pH 7.5/10 mM MgCl2/5mMDTT/0.2 mM ATP/0.5 *µ*Ci of [γ-32P]ATP) for 10 min at 30°C with 0.5 *µ*g of recombinant phosphatase inhibitor 2 (New England Biolabs). Control assays will also be carried out in the presence of the GSK-3 inhibitor Kenpaullone (2 *µ*M, Sigma-Aldrich). Reactions will be stopped by the addition of Laemmli loading buffer, boiled for 5 min, and resolved on SDS/10% PAGE. Gels will then be stained with Coomassie blue and autoradiographed. Incorporation of 32P into recombinant phosphatase inhibitor 2 will be measured by densitometry

### Analysis

All results obtained in this study will be quantified and analyzed by infrared spectrometry using a LiCor instrument.

Administration of a combination of cariprazine hydrochloride and escitalopram oxalate may synergistically stimulate phosphorylation of GSK3 in mouse brain, when compared to each compound administered alone.

## Claims

1. Trans-4-{2-[4-(2,3-dichlorophenyl)-piperazin-1-yl]-ethyl}-N,N-dimethylcarbamoyl-cyclohexylamine and pharmaceutically acceptable salts thereof for use in a method of treating major depressive disorder.

2. Trans-4-{2-[4-(2,3-dichlorophenyl)-piperazin-1-yl]-ethyl}-N,N-dimethylcarbamoyl-cyclohexylamine for use according to claim 1, in the form of trans-4-{2-[4-(2,3-dichlorophenyl)-piperazin-1-yl]-ethyl}-N,N-dimethylcarbamoyl-cyclohexylamine hydrochloride.

## Patentansprüche

1. Trans-4- {2- [4- (2,3-Dichlorphenyl)-piperazin-1-yl] -ethyl}-N, N-dimethylcarbamoylcyclohexylamin und pharmazeutisch annehmbare Salze davon zur Verwendung in einem Verfahren zur Behandlung von Major Depression.

2. Trans-4- {2- [4- (2,3-Dichlorphenyl) -piperazin-1-yl] -ethyl}-N, N-dimethylcarbamoylcyclohexylamin zur Verwendung nach Anspruch 1 in Form von trans-4- {2- [4- (2,3-Dichlorphenyl) -piperazin-1-yl] -ethyl} -N, N-dimethylcarbamoylcyclohexylamin-hydrochlorid.

## Revendications

1. Trans-4-{2-[4-(2,3-dichlorophényl)-pipérazin-1-yl]-éthyl}-N,N-diméthylcarbamoyl-cyclohexylamine et leurs sels pharmaceutiquement acceptables pour leur utilisation dans un procédé de traitement d'agents majeurs dépression.

2. Trans-4-{2-[4-(2,3-dichlorophényl)-pipérazin-1-yl]-éthyl}-N,N-diméthylcarbamoyl-cyclohexylamine pour leur utilisation selon la revendication 1, sous la forme de chlorhydrate de trans-4- {2-[4- (2,3-dichlorophényl) -pipérazin- 1-yl]- éthyl}-N,N-diméthylcarbamoyl-cyclohexylamine.
